# EUROPEAN PATENT SPECIFICATION

(11) **EP 1 433 445 B1**
(45) Date of publication and mention of the grant of the patent: **04.05.2005**
(21) Application number: 02425761.0
(22) Date of filing: 11.12.2002
(51) Int. Cl.: A61F 2/46, G01L 1/00, G01P 3/00

(54) **Apparatus for intraoperative measurement of the mechanical stability of an endoprosthesis implanted in a bone**
Vorrichtung zur intraoperativen Messung der mechanischen Festigkeit einer in einem Knochen implantierten Endoprothese
Dispositif pour la mesure intra-opératoire de la stabilité mécanique d'une endoprothèse implantée dans un os

(43) Date of publication of application: 30.06.2004
(73) Proprietor: UNIVERSITA DEGLI STUDI DI BOLOGNA, 40126 Bologna (IT); Istituti Ortopedici Rizzoli, 40136 Bologna (IT)
(72) Inventor: Cappello, Angelo, 40138 Bologna (IT); Cristofolini, Luca, 40125 Bologna (IT); Toni, Aldo, 40137 Bologna (IT); Viceconti, Marco, 40133 Bologna (IT)
(74) Representative: Lanzoni, Luciano

(56) References cited:
- EP-A- 0 290 375
- WO-A-90/06720
- DE-A- 3 639 263
- DE-B- 2 418 956
- GB-A- 2 259 452
- US-A- 4 922 898
- US-A- 5 320 625
- US-A1- 2002 115 944
- US-A1- 2002 143 268

## Description

The present invention refers to the surgical instrumentation designed to assist the surgeon in implanting some types of orthopedic prostheses which are fixed to the receptor bone without the aid of cement or other equivalent materials.

In particular, the present invention refers to an apparatus for the intraoperative measurement of a series of parameters indicating the degree of mechanical stability, in an implant site, of an orthopedic prosthesis which can be implanted in a bone by pressure insertion; a prosthesis in which the fixing stability is maintained by geometric interference and/or by forced coupling.

This information is important as it allows the surgeon to assess whether the force with which the prosthesis has been inserted in the bone is sufficient to ensure the success of the implant or whether, instead, the pressure must be increased until greater stability of the prosthesis with respect to the receptor bone is achieved.

Solutions to this problem have already been described by scientific literature in the form of devices designed to quantify this stability indirectly - not during surgery and not directly on the patient's body - but by means of laboratory experiments designed to simulate implant conditions in some way similar to the actual conditions of the implant. These laboratory experiments make it possible to provide the surgeon and/or the designer with general indications, the actual reproduction of which during the surgical operation depends totally on the skill, the sensitivity and the intuition of the surgeon.

It is known from document GB 2259452 a system designed to quantify this stability directly. The system and instrumentation disclosed provide a torque wrench adapter for the femoral rasp and for a hip stem implant. Each adapter includes a shaft for connecting a motion indicator thereon. The motion indicator includes a Port for accepting the shaft of either the rasp adapter or the implant adapter and an indicator needle which is linked through spring mechanisms to shiftable leg. In use, the leg is anchored at one end to the femur such that the leg moves with the femur and relative to the indicator housing which causes a deflection in the indicator needle to apprise the surgeon of small movement of either the rasp or the implant relative to the femur.

The aim of the present invention is to provide a solution to this problem by means of a device that can be used during surgery to measure the extent of the relative movement of the prosthesis with respect to the bone under the action of a certain load and to provide the orthopedic surgeon with corresponding quantitative information in real time; in other words that can provide the surgeon with indications as to the sufficiency of the degree of forcing of the prosthesis in the receptor bone site by measuring the slipping of the prosthesis caused by application of a certain test load.

According to the invention this aim is achieved by an apparatus for the intraoperative measurement of the mechanical stability of an orthopedic prosthesis according to one or more of the enclosed claims.

The technical features of the invention, with reference to the above aims, are clearly described in the claims below and its advantages are apparent from the detailed description which follows, with reference to the accompanying drawings which illustrate a preferred embodiment of the invention provided merely by way of example without restricting the scope of the inventive concept, and in which:
- Figure 1 is a perspective view of the apparatus, applied to a bone in which the prosthesis is implanted and shown during use;
- Figure 2 is a block diagram of information processing means with which the apparatus is equipped;

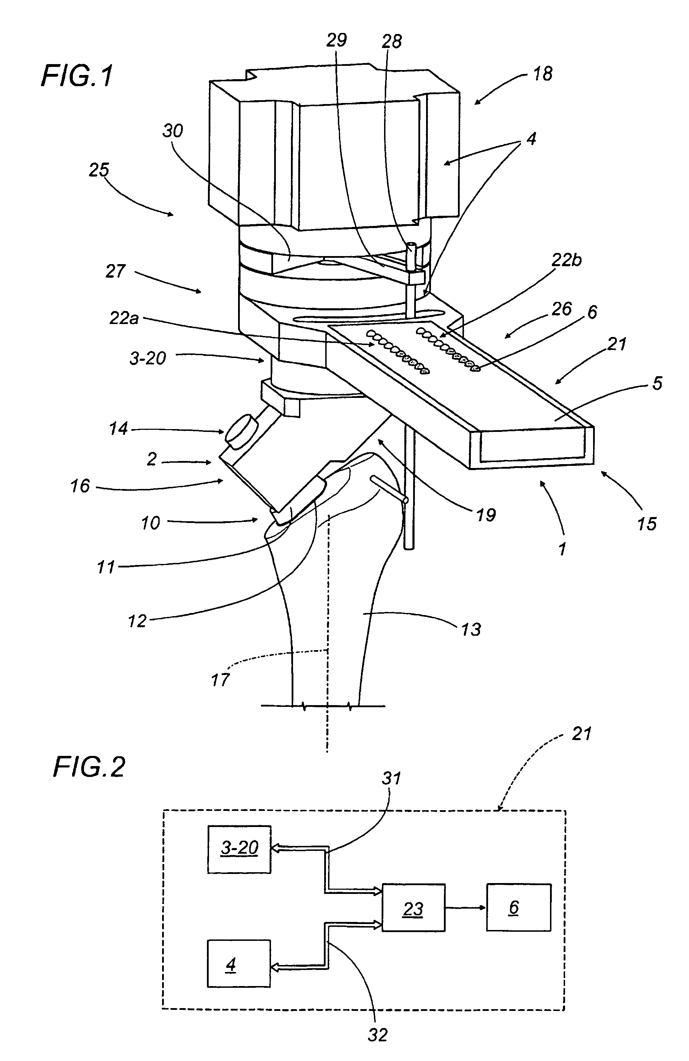

With reference to Figure 1, the numeral 25 denotes in its entirety an apparatus for measuring intraoperatively, in other words during a surgical operation and in real time, the mechanical stability of an orthopedic prosthesis 10 which can be implanted in a bone 13.

The apparatus 25 is described below with reference to the implantation of a hip prosthesis 10 having an anchoring element 11 which is implanted, by means of forced insertion, in a portion or a cavity 12 of the femoral bone of a patient. This application example should be intended as indicative and in no way restrictive since, as will become evident, the apparatus 25 can be used to ascertain the mechanical stability of the implantation of numerous other types of prostheses 10 currently used in the orthopedic field.

Whereas in the implant example of the prosthesis 10 described here the anchoring element 11 is fixed so that it protrudes from the cavity 12 of the bone with a collar 14 that projects externally from the receptor bone 13, and in other cases the prosthesis 10 is in any case provided with devices designed for rigid gripping (for example threaded holes or conical couplings), the mechanical stability of the implanted anchoring element 11 can be ascertained by the surgeon during the operation, by checking that this anchoring element 11 does not move with respect to the implant site under various load conditions applied in increasing order of intensity.

For this purpose the apparatus 25 is structured so as to essentially comprise means of application 2 of a mechanical test load to the anchoring element 11, appropriately measured by the element 3; and movement detection and measuring means 4 designed to detect the relative movement of the anchoring element 11 with respect to its site in the receptor bone 13.

The mechanical load application means 2 comprise a portable structure 15, which can be gripped by the surgeon or the operator, which is equipped with:
- gripping means 16 which allow rigid coupling (in the example, by tightening the collar 14, or, alternatively, by means of perforated flanges, threaded connectors, or other means) with the anchoring element 11 of the prosthesis 10;
- test load generation means 26 (in the example given here, the load is generated by the surgeon);
- and a mechanical transmission device 1 positioned between the gripping means 16 and the load generation means 26 (in the example given here, the same grip of the device 15). This mechanical transmission 1 is designed to transmit - to the anchoring element 11 - an output load, adequately proportional and corresponding to the input load received from and generated by the means of generation 26, in a remote location with respect to the anchoring element 11.

In particular, the portable structure 15 comprises two members 19 and 18, respectively proximal and distal to the anchoring element 11 of the prosthesis 10, which are mechanically constrained to each other so as to exchange forces and relative movements.

The proximal member 19 of the structure 15 provides support to the gripping means 16 which can be firmly and removably engaged with the collar 14 of the anchoring element 11.

The distal member 18, on the other hand, supports the load generation means 26.

The latter are preferably designed so as to work by exploiting the muscular strength of the surgeon or other operator. They therefore comprise a handgrip 5, on which the surgeon imparts a mechanical load and which is supported rotationally by the casing of the distal member 18.

The gripping means 16 and the handgrip 5, gripped by the surgeon or other operator, constitute respectively the output and input elements of the mechanical transmission 1 which foresees the interposition of a mechanical joint 27 between these elements.

The mechanical joint 27 is equipped with dynamometric means 20 designed to measure the load exerted by the surgeon, in other words the test load actually applied to the anchoring element 11 of the prosthesis 10.

The dynamometric means 20 can consist of numerous different embodiments, all functionally equivalent.

In the example described here, these dynamometric means 20 comprise a load cell 3 equipped with strain gauges, operatively positioned between the distal member 18 and the proximal member 19 of the mechanical transmission 1, in other words between the elements making up the mechanical joint 27.

The movement detection and measuring means 4 are designed to detect and measure relative slipping movements occurring between the anchoring element 11 and the implant cavity in the receptor bone 13.

These movement detection and measuring means 4 are associated with the relatively mobile members 18, 19 of the portable structure 15.

Since, in the application case of the apparatus described here, the most important movement to be prevented is represented by the possibility of slipping of the anchoring element 11 in the cavity 12 of the receptor bone 13, slipping being intended as the possibility of relative rotation around a longitudinal direction 17, these movement detection and measuring means 4 are designed to detect and measure such rotation, or in any case an associated rotation component, useful in quantifying for the surgeon the degree of stability of the anchoring element 11, as implanted up to that moment in the patient's bone. In general, the movement detection and measuring means 4 detect a relative movement (longitudinal, in rotation, or compound) between the prosthesis 10 and the receptor bone 13.

The movement detection and measuring means therefore comprise an angular movement transducer 4 which can have numerous embodiments, starting from the most elementary embodiment typically represented by a vernier scale angular movement transducer 4, up to the more advanced embodiment in which the angular movement transducer 4 consists of an encoder, as shown in figure 1.

In this case, the encoder works by detecting the relative rotation, under the test load, of the structure 15 of the apparatus 25 and of the receptor bone 13. A rod 28 associated with the bone 13 and connected to an arm 29, which in turn rotates in a guide 30 in the distal member 18 of the structure 15 of the apparatus 25, allows the encoder to detect the relative and actual slipping movements of the bone 13 with respect to the anchoring element 11, thus permitting the relative measurement.

The apparatus 25 also comprises processing means 21, which are interconnected with: the movement detection and measuring means 4; the dynamometric means 20; and with an output device 6.

The processing means 21 comprise - as can be seen in figure 2 - data acquisition channels 31, 32, connected to the movement detection and measuring means 4, to the applied mechanical load detection and measuring means 20 and to the processing unit 23 of these data.

The processing means 21 are therefore designed to exchange corresponding signals, to process them and to produce corresponding information on the output device 6.

In structural terms, in the example given here the processing means 21 are advantageously positioned inside the handgrip 5.

In this case, the output device 6 can also be integrated in the handgrip 5 and be embodied as LEDs 22a and 22b which are progressively activated with the variation in intensity of the parameter of significance for the surgeon.

In the case in question, therefore, a row of LEDs 22a provides the surgeon with an indication of the intensity of the test load applied; the other row of LEDs 22b, on the other hand, indicates the relative slipping effectively detected and measured between the anchoring element 11 of the prosthesis 10 and the cavity 12 of the receptor bone 13.

The LEDs 22a and 22b can be equipped with a measurement scale. Their progressive comparative lighting up provides the surgeon with an immediate visual assessment of the degree of stability achieved. Or, more simply, a single row of two-colour LEDs can simultaneously indicate the measurement of the applied load and whether the micromovement threshold has been exceeded.

The functioning of the apparatus 25 is clearly deducible from what is said above. It is appropriate here to simply point out that while performing the implant the surgeon can repeatedly make use of the apparatus 25 until the desired result is achieved.

In fact, after carrying out the forced insertion of the anchoring element 11 in the bone 13, the surgeon uses the apparatus 25 applied to the anchoring element 11 of the prosthesis 10, exerting a first test load on it. Having detected the measurement of the load effectively applied and the measurement of the slipping produced by this load, if the level of stability of the implant is found to be satisfactory the surgical operation is concluded in the traditional way; if on the other hand the stability is inadequate, the surgeon disengages the gripping means 16 of the apparatus 25, further forces the coupling between the anchoring element 11 and the bone 13 and then re-applies the apparatus 25, repeating the operations described above until the desired degree of stability is achieved.

The apparatus 25 described above provides an effective solution to the known technical problems discussed previously and allows the surgeon to perform quantitative and measurable assessments which can - if desired - be documented by means of recording instruments appropriately connected to the processing means 21. Such a systematic and rational working procedure ensures high-quality results and uniform repeatability of the results. As well as having advantageous repercussions on the rapidity of the surgical operation, this system also guarantees that the success of the implant - with respect to the known technique - depends to a much lesser extent on the skill and personal and subjective sensitivity of the surgeon.

The invention described can be subject to numerous modifications and variations without thereby departing from the scope of the inventive concept as defined by the appended claims. Moreover, all the details of the invention may be substituted by technically equivalent elements.

## Claims

1. An apparatus for intraoperative measurement of the mechanical stability of an orthopedic prosthesis (10) which can be implanted in or on a bone (13), in which the prosthesis (10) comprises an anchoring element (11) which can be implanted, by forcing, in a portion (12) of the receptor bone (13); the apparatus comprising:
- means of application (2) of a mechanical load, designed to apply an appropriately predetermined test load to the anchoring element (11) or to the relative receptor bone (13);
- movement detection and measuring means (4), to detect and measure relative slipping between the anchoring element (11) of the prosthesis and the receptor bone (13); said predetermined load being measured by dynamometric means (20); **characterised in that** the apparatus comprises
- processing means (21) which are connected to: the movement detection and measuring means (4); to the dynamometric means (20) and to an information output device (6), for exchanging corresponding signals, processing them and producing corresponding information on the output device (6).

2. The apparatus according to claim 1, in which the anchoring element (11) of the prosthesis (10) is equipped with a mechanical anchoring means (14), **characterised in that** the means of application (2) of the mechanical load comprise a portable structure (15) and gripping means (16), designed to combine with the mechanical anchoring means (14) of the anchoring element (11).

3. The apparatus according to claim 2, **characterised in that** the gripping means comprises a gripping device (16) which can be firmly and removably engaged with the anchoring means (14) of the anchoring element (11) of the prosthesis (10).

4. The apparatus according to claim 3, **characterised in that** the gripping device (16) comprises a series of jaws.

5. The apparatus according to claim 3, **characterised in that** the gripping device (16) comprises a perforated counter-flange.

6. The apparatus according to claim 1, **characterised in that** the relative slipping between the anchoring element (11) of the prosthesis and the portion (12) of the receptor bone (13) is implemented by a relative rotation between the anchoring element (11) and the cavity (12), the movement detection and measuring means (4) being designed to detect and measure at least one component of the relative motion.

7. The apparatus according to claim 1, **characterised in that** the relative slipping between the anchoring element (11) of the prosthesis and the portion (12) of the receptor bone (13) is implemented by a relative translation.

8. The apparatus according to claims 6 and 7, **characterised in that** the relative slipping between the anchoring element (11) of the prosthesis and the portion (12) of the receptor bone (13) is implemented by a combination of the relative translation and the relative rotation.

9. The apparatus according to claim 6 or 8, **characterised in that** the movement detection and measuring means comprise an angular movement transducer (4).

10. The apparatus according to claim 6 or 8, **characterised in that** the movement detection and measuring means comprise a linear movement transducer (4).

11. The apparatus according to claim 9 or 10, **characterised in that** the movement transducer (4) comprises a vernier scale.

12. The apparatus according to claim 9 or 10, **characterised in that** the movement transducer (4) comprises an encoder.

13. The apparatus according to claim 3, 6 or 7, **characterised in that** it comprises a mechanical transmission device (1) designed to transmit in output a predetermined load received in input to the mechanical load application means (2) associated with the anchoring element (11), in a remote position with respect to the anchoring element (11).

14. The apparatus according to claim 3, 6 or 7, **characterised in that** the movement detection and measuring means (4) are associated with members (18, 19) of the mechanical transmission, respectively distal and proximal to the anchoring element (11) of the prosthesis (10).

15. The apparatus according to claim 14, **characterised in that** the distal member (18) is equipped with a handgrip (5) which can be gripped by the surgeon, designed to receive and transmit a test load of muscular origin to the anchoring element (11) of the prosthesis (10).

16. The apparatus according to claim 13, **characterised in that said** dynamometric means (20) are associated with the mechanical transmission (1) to measure the test load applied to the anchoring element (11).

17. The apparatus according to claim 16, **characterised in that** the dynamometric means (20) comprise a load cell (3) positioned in use between the distal member (18) and the proximal member (19) of the mechanical transmission (1).

18. The apparatus according to claim 1, **characterised in that** the processing means (21) comprise data acquisition channels (31, 32) connected to the movement detection and measuring means and the dynamometric means (20); and a processing unit (23) for these data.

19. The apparatus according to claim 1, **characterised in that** the output device (6) comprises display devices (22a) which are progressively enabled with the variation in intensity of the detected relative slipping between the anchoring element (11) and the portion (12) of the receptor bone (13).

20. The apparatus according to claim 1, **characterised in that** the output device (6) comprises display devices (22b) which are progressively enabled with the variation in intensity of the test load applied to the anchoring element (11).

21. The apparatus according to claims 19 and 20, **characterised in that** one or each display device is embodied as a series of differentially enabled LEDs (22a, 22b).

22. The apparatus according to any of the foregoing claims, **characterised in that** the prosthesis (10) is an orthopedic type prosthesis (10), the anchoring element (11) being implanted in a receptor bone (13) of the patient.

## Patentansprüche

1. Vorrichtung zur interaktiven Messung der mechanischen Festigkeit einer in einem Knochen implantierten Endoprothese (10), welche in oder an einem Knochen (13) implantiert werden kann, und bei welcher die Prothese (10) ein Verankerungselement (11) enthält, welches durch Einpressen in einem Abschnitt (12) des Aufnahmeknochens (13) implantiert werden kann, wobei die Vorrichtung enthält:
- Anbringungsmittel (2) einer mechanischen Belastung, dazu bestimmt, an dem Verankerungselement (11) oder an dem entsprechenden Aufnahmeknochen (13) eine geeignet festgelegte Testbelastung anzubringen;
- Bewegungserfassungs- und Messmittel (4), um ein entsprechenden Gleiten zwischen dem Verankerungselement (11) der Prothese und dem Aufnahmeknochen (13) zu erfassen und zu messen; wobei die genannte festgelegte Belastung durch dynamometrische Mittel (20) gemessen wird; **dadurch gekennzeichnet, dass** die Vorrichtung enthält:
- Verarbeitungsmittel (21), welche angeschlossen sind an: die Bewegungserfassungs- und Messmittel (4); die dynamometrischen Mittel (20) und an eine Ausgangsvorrichtung (6) der Informationen, und zwar zum Austausch von entsprechenden Signalen, zur Verarbeitung derselben und zum Erzeugen entsprechender Informationen an der Ausgangsvorrichtung (6).

2. Vorrichtung nach Patentanspruch 1, bei welcher das Verankerungselement (11) der Prothese (10) mit mechanischen Verankerungsmitteln (14) versehen ist, **dadurch gekennzeichnet, dass** die Anbringungsmittel (2) der mechanischen Belastung eine tragbare Struktur (15) und Spannmittel (16) enthalten, dazu bestimmt, mit den mechanischen Verankerungsmitteln (14) des Verankerungselementes (11) zusammenzuwirken.

3. Vorrichtung nach Patentanspruch 2, **dadurch gekennzeichnet, dass** die Spannmittel eine Spannvorrichtung (16) enthalten, welche fest und lösbar mit den Verankerungsmitteln (14) des Verankerungselementes (11) der Prothese (10) verbunden werden kann.

4. Vorrichtung nach Patentanspruch 3, **dadurch gekennzeichnet, dass** die Spannvorrichtung (16) eine Serie von Klemmbacken enthält.

5. Vorrichtung nach Patentanspruch 3, **dadurch gekennzeichnet, dass** die Spannvorrichtung (16) einen perforierten Gegenflansch enthält.

6. Vorrichtung nach Patentanspruch 1, **dadurch gekennzeichnet, dass** das entsprechende Gleiten zwischen dem Verankerungselement (11) der Prothese und dem Abschnitt (12) des Aufnahmeknochens (13) durch eine entsprechende Umdrehung zwischen dem Verankerungselement (11) und der Höhlung (12) erfolgt, wobei die Bewegungserfassungs- und Messmittel (4) dazu bestimmt sind, wenigstens eine Komponente der entsprechenden Bewegung zu erfassen und zu messen.

7. Vorrichtung nach Patentanspruch 1, **dadurch gekennzeichnet, dass** das entsprechende Gleiten zwischen dem Verankerungselement (11) der Prothese und dem Abschnitt (12) des Aufnahmeknochens (13) durch eine entsprechende Translationsbewegung erfolgt.

8. Vorrichtung nach den Patentansprüchen 6 und 7, **dadurch gekennzeichnet, dass** das entsprechenden Gleiten zwischen dem Verankerungselement (11) der Prothese und dem Abschnitt (12) des Aufnahmeknochens (13) durch eine Kombination der entsprechenden Translationsbewegung und der entsprechenden Umdrehung erfolgt.

9. Vorrichtung nach Patentanspruch 6 oder 8, **dadurch gekennzeichnet, dass** die Bewegungserfassungs- und Messmittel einen Winkelbewegungswandler (4) enthalten.

10. Vorrichtung nach Patentanspruch 6 oder 8, **dadurch gekennzeichnet, dass** die Bewegungserfassungs- und Messmittel einen Linearbewegungswandler (4) enthalten.

11. Vorrichtung nach Patentanspruch 9 oder 10, **dadurch gekennzeichnet, dass** der Bewegungswandler (4) einen Nonius enthält.

12. Vorrichtung nach Patentanspruch 9 oder 10, **dadurch gekennzeichnet, dass** der Bewegungswandler (4) einen Encoder enthält.

13. Vorrichtung nach Patentanspruch 3, 6 oder 7, **dadurch gekennzeichnet, dass** sie eine mechanische Übertragungsvorrichtung (1) enthält, dazu bestimmt, im Ausgang eine bestimmte Belastung auf die Anbringungsmittel (2) der mechanischen Belastung zu übertragen, welche dem Verankerungselement (11) zugeordnet sind, erhalten im Eingang in einer entfernten Position im Verhältnis zu dem Verankerungselement (11).

14. Vorrichtung nach Patentanspruch 3, 6 oder 7, **dadurch gekennzeichnet, dass** die Bewegungserfassungs- und Messmittel (4) Elementen (18, 19) der mechanischen Übertragung zugeordnet sind, jeweils distal oder proximal zu dem Verankerungselement (11) der Prothese (10).

15. Vorrichtung nach Patentanspruch 14, **dadurch gekennzeichnet, dass** das distale Element (18) mit einem Handgriff (5) versehen ist, welcher von dem Chirurgen gegriffen werden kann und dazu dient, eine Testbelastung muskulären Ursprungs zu empfangen und auf das Verankerungselement (11) der Prothese (10) zu übertragen.

16. Vorrichtung nach Patentanspruch 13, **dadurch gekennzeichnet, dass** die genannten dynamometrischen Mittel (20) der mechanischen Übertragung (1) zugeordnet sind, um die auf das Verankerungselement (11) ausgeübte Testbelastung zu messen.

17. Vorrichtung nach Patentanspruch 16, **dadurch gekennzeichnet, dass** die dynamometrischen Mittel (20) eine Ladezelle (3) enthalten, die betrieblich zwischen dem distalen Element (18) und dem proximalen Element (19) der mechanischen Übertragung (1) positioniert ist.

18. Vorrichtung nach Patentanspruch 1, **dadurch gekennzeichnet, dass** die Verarbeitungsmittel (21) Datenerfassungskanäle (31, 32) enthalten, angeschlossen an die Bewegungserfassungs- und Messmittel und an die dynamometrischen Mittel (20); sowie eine Verarbeitungseinheit (23) für diese Daten.

19. Vorrichtung nach Patentanspruch 1, **dadurch gekennzeichnet, dass** die Ausgangsvorrichtung (6) Display-Vorrichtungen (22a) enthält, die mit der Veränderung der Intensität des erfassten entsprechenden Gleitens zwischen dem Verankerungselement (11) und dem Abschnitt (12) des Aufnahmeknochens (13) progressiv befähigt werden.

20. Vorrichtung nach Patentanspruch 1, **dadurch gekennzeichnet, dass** die Ausgangsvorrichtung (6) Display-Vorrichtungen (22b) enthält, die mit der Veränderung der Intensität der auf das Verankerungselement (11) ausgeübten Testbelastung progressiv befähigt werden.

21. Vorrichtung nach den Patentansprüchen 19 und 20, **dadurch gekennzeichnet, dass** eine oder jede DisplayVorrichtung als eine Serie von differenziert befähigten LED's (22a, 22b) ausgeführt ist.

22. Vorrichtung nach einem beliebigen der vorstehenden Patentansprüche, **dadurch gekennzeichnet, dass** die Prothese (10) eine orthopädische Prothese (10) ist, wobei das Verankerungselement (11) in einen Aufnahmeknochen (13) des Patienten implantiert wird.

## Revendications

1. Un dispositif pour la mesure intra-opératoire de la stabilité mécanique d'une prothèse orthopédique (10) qui peut être implantée dans ou sur un os (13), dans lequel la prothèse (10) comprend un élément d'ancrage (11) qui peut être implanté, par forçage, dans une portion (12) de l'os récepteur (13) ; le dispositif comprenant :
- des moyens (2) d'application d'une charge mécanique, destinés à appliquer une charge d'essai opportunément prédéterminée à l'élément d'ancrage (11) ou à l'os récepteur (13) correspondant ;
- des moyens (4) de détection et de mesure de déplacement, destinés à détecter et à mesurer un glissement relatif entre l'élément d'ancrage (11) de la prothèse et l'os récepteur (13) ; ladite charge prédéterminée étant mesurée par des moyens dynamométriques (20) ; ledit dispositif étant **caractérisé en ce qu'**il comprend également
- des moyens de traitement (21) qui sont reliés : aux moyens (4) de détection et de mesure du déplacement ; aux moyens dynamométriques (20) et à un dispositif de sortie (6) des informations, pour échanger des signaux correspondants, les traiter et produire des informations correspondantes sur le dispositif de sortie (6).

2. Le dispositif selon la revendication 1, dans lequel l'élément d'ancrage (11) de la prothèse (10) est pourvu d'un moyen d'ancrage mécanique (14), **caractérisé en ce que** les moyens (2) d'application de la charge mécanique comprennent une structure portable (15) et des moyens de prise (16) destinés à s'associer avec le moyen d'ancrage mécanique (14) de l'élément d'ancrage (11).

3. Le dispositif selon la revendication 2, **caractérisé en ce que** les moyens de prise comprennent un dispositif de prise (16) qui peut être associé, fermement et de façon amovible, au moyen d'ancrage (14) de l'élément d'ancrage (11) de la prothèse (10).

4. Le dispositif selon la revendication 3, **caractérisé en ce que** le dispositif de prise (16) comprend une série de mâchoires.

5. Le dispositif selon la revendication 3, **caractérisé en ce que** le dispositif de prise (16) comprend une contre-bride perforée.

6. Le dispositif selon la revendication 1, **caractérisé en ce que** le glissement relatif entre l'élément d'ancrage (11) de la prothèse et la portion (12) de l'os récepteur (13) est mis en oeuvre par une rotation relative entre l'élément d'ancrage (11) et la cavité (12), les moyens (4) de détection et de mesure du déplacement étant destinés à détecter et à mesurer au moins une composante du déplacement relatif.

7. Le dispositif selon la revendication 1, **caractérisé en ce que** le glissement relatif entre l'élément d'ancrage (11) de la prothèse et la portion (12) de l'os récepteur (13) est mis en oeuvre par une translation relative.

8. Le dispositif selon les revendications 6 et 7, **caractérisé en ce que** le glissement relatif entre l'élément d'ancrage (11) de la prothèse et la portion (12) de l'os récepteur (13) est mis en oeuvre par une combinaison de la translation relative et de la rotation relative.

9. Le dispositif selon la revendication 6 ou 8, **caractérisé en ce que** les moyens de détection et de mesure du déplacement comprennent un transducteur (4) de déplacement angulaire.

10. Le dispositif selon la revendication 6 ou 8, **caractérisé en ce que** les moyens de détection et de mesure du déplacement comprennent un transducteur (4) de déplacement linéaire.

11. Le dispositif selon la revendication 9 ou 10, **caractérisé en ce que** le transducteur de déplacement (4) comprend une échelle de vernier.

12. Le dispositif selon la revendication 9 ou 10, **caractérisé en ce que** le transducteur de déplacement (4) comprend un encodeur.

13. Le dispositif selon la revendication 3, 6 ou 7, **caractérisé en ce qu'**il comprend un dispositif de transmission mécanique (1) destiné à transmettre en sortie une charge prédéterminée, reçue en entrée, aux moyens (2) d'application de la charge mécanique associés à l'élément d'ancrage (11), dans une position distante par rapport à l'élément d'ancrage (11).

14. Le dispositif selon la revendication 3, 6 ou 7, **caractérisé en ce que** les moyens (4) de détection et de mesure du déplacement sont associés à des membres (18, 19) de la transmission mécanique, respectivement distal et proximal à l'élément d'ancrage (11) de la prothèse (10).

15. Le dispositif selon la revendication 14, **caractérisé en ce que** le membre distal (18) est pourvu d'une poignée (5) pouvant être empoignée par le chirurgien et destinée à recevoir et à transmettre une charge d'essai d'origine musculaire à l'élément d'ancrage (11) de la prothèse (10).

16. Le dispositif selon la revendication 13, **caractérisé en ce que** lesdits moyens dynamométriques (20) sont associés à la transmission mécanique (1) afin de mesurer la charge d'essai appliquée à l'élément d'ancrage (11).

17. Le dispositif selon la revendication 16, **caractérisé en ce que** les moyens dynamométriques (20) comprennent une cellule de charge (3) positionnée, durant l'utilisation, entre le membre distal (18) et le membre proximal (19) de la transmission mécanique (1).

18. Le dispositif selon la revendication 1, **caractérisé en ce que** les moyens de traitement (21) comprennent des canaux (31, 32) d'acquisition de données, reliés aux moyens de détection et de mesure du déplacement et aux moyens dynamométriques (20) ; ainsi qu'une unité de traitement (23) pour le traitement de ces données.

19. Le dispositif selon la revendication 1, **caractérisé en ce que** le dispositif de sortie (6) comprend des dispositifs de visualisation (22a) qui sont progressivement activés au fur et à mesure que varie l'intensité du glissement relatif détecté entre l'élément d'ancrage (11) et la portion (12) de l'os récepteur (13).

20. Le dispositif selon la revendication 1, **caractérisé en ce que** le dispositif de sortie (6) comprend des dispositifs de visualisation (22b) qui sont progressivement activés au fur et à mesure que varie l'intensité de la charge d'essai appliquée à l'élément d'ancrage (11).

21. Le dispositif selon les revendications 19 et 20, **caractérisé en ce qu'**un ou chaque dispositif de visualisation est réalisé sous forme d'une série de DEL (22a, 22b) à activation différentielle.

22. Le dispositif selon l'une quelconque des revendications précédentes, **caractérisé en ce que** la prothèse (10) est une prothèse (10) de type orthopédique, l'élément d'ancrage (11) étant implanté dans un os récepteur (13) du patient.
